# EUROPEAN PATENT SPECIFICATION

(11) **EP 3 790 505 B1**
(45) Date of publication and mention of the grant of the patent: **19.02.2025**
(21) Application number: 19730514.7
(22) Date of filing: 07.06.2019
(51) Int. Cl.: A61M 1/36

(54) **VASCULAR ACCESS TUBE**
SCHLAUCH FÜR VASKULÄREN ZUGANG
TUBE D'ACCÈS VASCULAIRE

(30) Priority: 29.06.2018 GB 201810689
(43) Date of publication of application: 17.03.2021
(73) Proprietor: Spectrum Medical Ltd., Gloucester GL2 9QL (GB)
(72) Inventor: TAMARI, Jeremy, Cheltenham Road East Gloucester Gloucestershire GL2 9QL (GB)
(74) Representative: Marks & Clerk LLP
(86) International application number: PCT/GB2019/051586
(87) International publication number: WO 2020/002872

(56) References cited:
- WO-A2-2011/112755
- US-A- 6 090 067
- US-B2- 6 663 590

## Description

### Field of the Invention

The present invention relates to a vascular access tube, in particular a tube used as an arterial access graft. The present invention also relates to an assembly using such a vascular access tube. In an exemplary application the invention is used for removing blood from a left ventricular cavity.

### Background

During extracorporeal ventilation techniques, such as venous-arterial extracorporeal membrane oxygenation (ECMO) or left ventricular assist procedures, a problem can arise when blood accumulates in a heart chamber, in particular in the left ventricular cavity.

The left ventricle is the thickest muscle in the heart and, in a healthy person, is expected to carry out most of the "pumping" work of any heart tissue. The left ventricle also requires itself a large blood supply to meet its metabolic demands. If the left ventricle fails, then a treatment involves reducing the amount of pumping work demanded from the left ventricle and supplying oxygenated blood to the left ventricle, to support and/or revive the left ventricle. This procedure is called a "left ventricle assist" or "left heart bypass".

The blood flow fed towards the ventricular muscle is determined by the pressure difference between the aorta and the ventricular wall. If blood is trapped in the left ventricular cavity, this reduces the pressure differential and, as a consequence, blood flow through the heart muscle is reduced.

WO 2011/112755 A2 relates to a self-closing device for implantation within a patient's body during dialysis, the device including a base material and a plurality of support elements surrounding or embedded in the base material. US 6663590 B2 relates to vascular access systems and devices for facilitating repeated access to a blood vessel for external treatment of blood, such as dialysis. US 6090067 A relates to a surface access haemostatic valve for use during dialysis, the valve comprising a tube and a haemostatic valve mounted at the distal end for positioning at the surface of the skin of the patient.

The present invention seeks to provide a system that facilitates removal of blood from the left ventricle.

### Summary of the Invention

The invention is defined by the features of independent claim 1.

According to a first aspect of the invention, there is provided an arterial access graft for blood subject to a driving pressure, as defined in claim 1. The arterial access graft comprises a wall defining a main lumen, wherein a portion of the wall comprises an access port for an object to be introduced into the tube through the wall, wherein the access port comprises a biasing structure providing a self-closing behaviour sufficiently strong to remain fluid-tight when exposed to a driving pressure of up to 200 mmHg (0.263 atm), whereby the access port is sufficiently fluid-tight to contain pressurised fluid flowing through the vascular access tube.

A vascular access tube may be a so-called "graft" such as an arterial access tube used for arterial anastomosis (surgical attachment). As the skilled person will appreciate, the wall defining the lumen of the vascular access tube has a tubular structure permitting blood flow through the tube. To contain blood pumped therethrough, such access tubes are capable of withstanding driving pressures that will be described in more detail below.

An arterial access tube may be used to access the axillary artery, for example the right axillary artery. The right axillary artery is connected to the aorta and therefore the aortic valve, next to which is the left ventricle. By accessing the axillary artery, the vascular access tube can be used as a port for a catheter or other suitable device requiring access into the axillary artery and optional extension across the aortic valve and into the left ventricle. A catheter into the left ventricle facilitates removing fluid, particularly blood during a procedure commonly known as a "left ventricular assist", left ventricular support, A-V support, A-V ECMO, or cardiac bypass procedure.

An exemplary use for the vascular access tube may be as an access site to a coronary artery for the provision of one or more coronary interventions, such as stents. Alternatively, or in addition, the vascular access tube may be used to access vasculature in order to provide an imaging agent, such as a dye, to the vasculature. The vasculature or parts of it can then quickly and easily be imaged, for instance in order to ascertain the presence or absence of abnormalities. However, it may not be possible to gain access to the lumen (inside the tube) after the tube has been implanted. The present invention facilitates access to the lumen through the tube wall.

The object to be introduced may be a catheter to be introduced laterally through the wall of the graft. By "lateral" introduction, it is meant that an object is inserted or introduced into the lumen through the wall of the tube, rather than through an opening at either end of the tube.

It may be undesirable to pierce the graft wall as this would cause a hole in the graft wall from where pressurised blood would uncontrollably spray. The purpose of the self-closing access port is to provide a structure in the wall through which an object, such as a catheter, may be inserted into the tube's main lumen and which allows removal of the external object, while maintaining a fluid-tight channel through the tube.

The self-closing behaviour is sufficiently strong to maintain the fluid-tight condition when the access port is penetrated by introduction of said object to be provided and sufficiently strong to maintain the fluid-tight condition after removal of the object.

By sufficiently fluid-tight, it is understood that the seal is capable of containing fluid such as blood flowing through a blood vessel system at blood driving pressures and at blood flow rates typically observed in patients. Such pressures vary according to several circumstances, including vessel type and location, patient posture and height. Pressure differences of less than 10 mmHg may suffice to drive blood in the region of usually up to 5 litres per minute, and driving pressures may be in the region of low 100 mmHg blood pressure (i.e. above atmospheric pressure; 1 atm = 760 mmHg). Driving pressures can be lower during routine use but the pressure observable at the tube walls may temporarily increase. For instance, the pressure may be higher in the case of an occlusion.

In this regard, blood may be pumped through an arterial access graft at driving pressures in the region of 200 mmHg (above atmospheric pressure). In the absence of heart activity, the driving pressure may be provided by an external flow control device such as a roller pump or a centrifugal pump.

In the tube lumen, pressures may increase in the case of an occlusion, when a flow control device (pump) continues to pump until the occlusion is removed, or until the pump is turned down. In that case, the intraluminal pressure may temporarily increase, and may approach the driving pressure of the external flow control device, for instance the driving pressure may reach 200 mmHg or 350 mmHg. Higher values would not normally be expected. An access port able to remain self-sealed at luminal fluid pressure of up to 200 mmHg or up to 350mmHg will provide a practically fluid-tight configuration in an arterial access graft, withstanding even a temporary increase in pressure. It is believed that an arrangement withstanding driving pressures that are lower than that, e.g. 50 mmHg, may suffice in some scenarios e.g. for testing purposes.

To provide an illustration of scale, an arterial access graft may have a diameter of around 8 mm and a catheter to be introduced may have in the region of 8 "French" or 2.66 mm (1 mm = 3 French). A 2.3 mm hole pierced into the side wall of an 8 mm diameter tube would constitute a significant puncture. The access port provides a structure capable not only of self-sealing the hole of that size, but also to maintain the access port fluid-tight at driving pressures of up to 200 mmHg. Designs may include access ports that remain fluid-tight at driving pressures of up to 50 mmHg, up to 100 mmHg, up to 150 mmHg, up to 200 mmHg, up to 250 mmHg, up to 300 mmHg, up to 350 mmHg, or up to 400 mmHg.

The biasing structure may have a self-closing behaviour that is sufficiently strong with reference to the driving performance of a blood circulation pump to be provided. For instance, an exemplary maximum driving pressure of a blood circulation pump may be 350 mmHg. The biasing structure may be dimensioned to withstand 350 mmHg driving pressure.

The sealability/accessibility properties of the biasing structure provide a reversibly closable structure that can maintain a fluid-tight seal of the main lumen against the exterior of the access tube before, during and after penetration by an object to be provided, i.e. before introduction of an object, while the object is located through the fluid-tight seal and after the object has been removed. The access port provides an "on-demand" access into the lumen, as and when required.

The biasing structure may be understood as being a self-closing mechanism. By "self-closing", it will be appreciated that if an aperture is formed or is present in the self-closing structure, the self-closing structure is capable of sealing, closing or contracting the aperture, and/or capable of forming a boundary with an object inserted through the aperture such that the structure remains fluid-tight. A skilled person will have no difficulty identifying a self-closing structure capable of containing fluid with a driving pressure of up to 50 mmHg or higher.

In some embodiments, biasing structure comprises a self-sealing membrane.

A self-sealing membrane may be provided initially without an opening and, once pierced at a location, will close to seal at the location to be practically as fluid-tight as if the membrane had not been pierced. Suitable self-sealing membranes, such as haemostatic self-sealing membranes, are known to a skilled person.

In some embodiments, the biasing structure comprises a valve.

The access port may comprise an aperture already present in the structure, such as a slit valve or duck bill valve. The biasing structure may be provided by a valve oriented such that it is urged into a fluid-tight condition by pressurised fluid. The access port comprises a biasing structure providing a self-closing behaviour may be constituted by a haemostatic valve.

In some embodiments, the self-closing behaviour of the biasing structure is stronger than of the wall.

It will be appreciated that the access port may have, by way of the biasing structure, a stronger self-closing behaviour than remaining portions of the wall without an access port. To put this in context, the remainder of the wall, such as a graft, may be manufactured with properties other than resealability in mind, for instance for biocompatibility purposes or stability requirements, which may inherently provide less self-sealing capability. By this is meant that an aperture is formed in the remainder of the wall, the remainder of the wall is less capable of closing the aperture.

The stronger self-closing behaviour may be provided by a seal or valve structure that is thicker that the main lumen wall. The stronger self-closing behaviour may be provided by a seal or valve structure that has stronger resiliently deformable properties that the main lumen wall.

In some embodiments, the access port comprises an access arm comprising an access lumen, the access arm extending from the wall.

In the context of the present invention, the term "access arm" will be understood to mean a tube having a lumen which projects from the vascular access tube. The access lumen joins into the main lumen. The seal that may be located at the wall-joining end of the access arm and/or by a seal part way along the access arm or at the distal end (the end opposite the wall-joining end).

By providing a seal in an access arm, this facilitates the incorporation of different seal types at the product design stage for otherwise unmodified graft structures. For instance, a seal of different thickness with different self-sealing strength can be provided in the access arm if required, without this affecting the structure of the main lumen wall. The access arm facilitates guiding an object, for example a catheter, into the vascular access tube.

It will be appreciated that the access arm will have a first end and a second end. One of the ends is located distal to the lumen of the vascular access tube, i.e. a free end. One end joins the vascular access tube.

The access arm may be integral with the vascular access tube, for instance a single-piece moulded or woven structure. This will be understood to mean that the access arm and the vascular access tube are formed as one component rather than the joining together of separate components. The vascular access tube may be provided in the form of a "Y" or "T" junction of which one arm is sealed by the access port and the other two arms provide the main lumen of the vascular access tube. In other embodiments the access arm may be a separate component to the vascular access tube that are joined, for example sewn, glued or otherwise bound together. Regardless of whether the access arm and the vascular access tube are integral or joined, the access arm and the vascular access tube provide a structure merging the main lumen and the access lumen, allowing, in the absence of occlusions, fluid communication with the lumen of the vascular access tube.

In some embodiments, the access arm is formed of the same material as the wall of the vascular access tube. In other embodiments, the access arm is formed of different material to the wall of the vascular access tube.

It will be understood that the vascular access tube has a first end and a second end. In some embodiments, the access port comprises a portion of the wall of the vascular access tube which is closer to the first end than to the second end of the vascular access tube i.e. the access point is off-centre. In some embodiments the access port comprises a portion of the wall of the vascular access tube between the mid-point and the first end of the vascular access tube. In some embodiments the access port comprises a portion of the wall between the mid-point and the second end of the vascular access tube. The access port may comprise a portion of the wall in a proximal or distal quarter of the tube, for instance nearer to the second end than the mid-point of the vascular access tube. Alternatively, the access port may comprise a portion of the tubular wall nearer to the first end than the mid-point of the vascular access tube. Positioning the access port off-centre may improve ease of access to the port and thus to the patient.

In particular, it is envisaged that, in use, when the vascular access tube is implanted in the patient, at least the portion of the wall comprising the access port, or in embodiments comprising the access arm, at least the end of the access arm distal to the lumen of the vascular access tube, is sufficiently long so that its length extends outside a patient. This facilitates access via the access port to the patient's vasculature.

The access arm increases the distance an object, such as a catheter, has to be pushed before entering the main lumen, which reduces the risk of damaging the inner main lumen wall opposite the access port when a sharp object is pushed through the access port with force.

The access arm facilitates gripping the access tube where the access port is. If it is imagined how this might work in practice, pushing a sharp object through a curved outer wall requires more skill than pushing a sharp object along the axis of a tube, and so the provision of an access arm reduces the risk of not piercing the access port as intended.

The access arm facilities closing the access port by clamping, pinching, or sewing it up if it is desired to close the access port permanently.

The access arm may be longer than, of equal length, or shorter than one or both of its adjacent tube portions of the vascular access tube.

In some embodiments, the lumen of the access arm has a smaller diameter than the lumen of the vascular access tube. This assists in guiding an object which has a smaller diameter than the lumen of the vascular access tube, for example a stent or catheter.

The diameter of the lumen of the vascular access tube is configured such that the tube can be connected or inserted into a patient's vasculature. Suitable diameters will be known to the skilled person.

In some embodiments the lumen of the vascular access tube has a diameter of at least 3mm, 4mm, 5mm, or 6mm, and/or no more than 8mm, 9mm, 10mm, 11mm, or 12mm.

The lumen of the access arm may have a diameter of less than 12mm, less than 10mm, less than 9mm, less than 8mm, less than 7mm, less than 6mm, less than 5mm, less than 4mm, less than 3mm or less than 2mm.

In some embodiments, the biasing structure is positioned to seal an end of the access arm distal to the main lumen of the vascular access tube.

The seal structure may be positioned close to or at the distal end of the access arm. Alternatively, the seal structure may be close to or at the wall-joining end of the access arm, i.e. the end closer to the vascular access tube. A plurality of seal structures may be provided in the access arm. For instance, a seal structure may be provided near the distal end and a seal structure near the proximal end. A plurality of reversibly closable structures in series provides fail-safe closures in case one seal fails.

The access arm may extend at an included angle of at least 10°, at least 20°, at least 30°, at least 40°, at least 45°, at least 50°, at least 60°, at least 70°, at least 80°, at least 90°, at least 100°, at least 110°, at least 120°, at least 130°, at least 140°, at least 150°, at least 160° or at least 170° from the tubular wall of the vascular access tube. It will be understood that the included angle is specified as being measured from the first end of the vascular access tube.

In some embodiments, the access arm extends at an angle of no more than 10°, no more than 20°, no more than 30°, no more than 40°, no more than 45°, no more than 50°, no more than 60°, no more than 70°, no more than 80°, no more than 90° from the tubular wall of the vascular access tube.

In embodiments where the access arm extends at an angle of approximately 90° from the wall of the vascular access tube, it will be understood that the access arm is substantially perpendicular to the axis of the lumen of the vascular access tube.

It will be appreciated that tubes/arms of the present invention are flexible, for instance due to the use of a flexible material or flexible wall structure, and so can be adjusted to follow the curves and lines of the patient's vasculature without damaging the vasculature wall.

In some embodiments, the flexible material consists or comprises a polymer or copolymer, for instance polytetrafluoroethylene (Teflon or PTFE), polyethylene terephthalate (Dacron) or polyurethane. The polytetrafluoroethylene may be expanded polytetrafluoroethylene (ePTFE).

In some embodiments, the flexible material comprises or consists of material formed of fibres. For instance, the flexible material may be woven fibrous material. The fibres may be artificial or natural. An example of a woven artificial fibre is dacron. Another woven artificial fibre is polyurethane. Other suitable materials will be known to a person skilled in the art.

One or more additional materials may be embedded or coated into/onto the material which forms the wall of the vascular access tube and/or the access arm. For instance, the interior of the tube (i.e. the surface of the tube facing the lumen) may be coated with an additional material. Alternatively or in addition to, the exterior of the tube (i.e. the surface of the tube external to the lumen) may be coated with an additional material. The additional material may comprise or consist of PTFE, ePTFE, dacron, polyurethane, or any combination thereof.

The additional material may comprise or consist of an antimicrobial material such as an anti-microbial metal. Example antimicrobial metals include gold, silver or copper. The additional material may be the same for the outer coating and for the inner coating. The additional material may be different for the outer coating and for the inner coating.

In some embodiments, the main lumen comprises a partition structure extending along at least a portion of the length of the tube to separate the main lumen into at least two passages.

In some embodiments, the cross-section (perpendicular to the tube axis) of one of the passages is greater than another passage.

It will be appreciated that, by way of the partition structure, the first and the second passage are fluidically isolated from each other. The partition wall may extend along at least or portion of the length of the vascular access tube. In this way, the portion of the entire lumen of the vascular access tube is partitioned into a first and second passage. Multiple partitions may be provided to separate the lumen into multiple passages.

In some embodiments, the access port joins into one passage.

The access port may be positioned such that it provides access into only one of the passages. Thereby, one passage is accessible via the access port and the other passage is fluidically isolated by the partition structure from the access port. It will be understood that the other passage remains accessible via the first or second end of the vascular access tube.

A partition structure may assist in separating blood flow in a first passage from an inserted object or device in the second passage, such as a catheter or stent.

A partition structure may also provide the function of a guide channel and/or scaffold to assist in guiding and/or supporting an inserted object through the vascular access tube.

In some embodiments, the cross-section (i.e. the diameter for a round tube) of the first passage may be greater than the cross-section of the second passage.

The internal partition structure may be collapsible. For instance, the internal partition structure may have a pleated or folded structure, such that the structure can be expanded by stretching the pleats/folds, or collapsing by allowing the pleats/folds to form. Likewise, the partition structure may be longer than is necessary to span the direct wall-to-wall contact distance between its wall contact points. The partition structure may be sufficiently long to allow it to lie circumferentially against the inner lumen circumference from wall-to-wall contact. If only one of the passages is supplied with pressurised fluid, the partition structure would be expected to bow/collapse in the direction of the other passage in the absence of a supporting structure (such as a catheter if this is inserted through a passage). Thus, if the second passage is empty, the first passage is expandable to practically the full cross-section of the main lumen.

In some embodiments the internal partition structure comprises or consists of a flexible material. Example flexible materials have been described elsewhere herein.

In some embodiments, the vascular access tube is comprised in a blood removal assembly suitable for removing blood from a left ventricular cavity as defined in claim 11. The assembly comprises, in addition to the vascular access tube, a catheter dimensioned to fit through the access port and of sufficient length to extend from outside the access port beyond and end the vascular access tube.

As set out above, the vascular access tube may be an arterial access graft. A typical diameter of an arterial access graft is in the region of 8 mm. The catheter diameter may be in the region of about 8 French (2.66 mm). The access port may have a diameter somewhat larger than the catheter diameter, in the region of 10-12 French (3.33 to 4 mm), and is therefore dimensioned to accommodate the catheter.

The length of the catheter may be considerably longer than the vascular access tube, so that it is suitable for reaching the left ventricular cavity.

A catheter will be understood to be a tube suitable for insertion into a subject. The tube is suitable for removing and/or introducing fluid from and/or into the subject. It is therefore envisaged that catheter be used for removing stagnant and/or clotted blood from the left ventricular cavity.

In some embodiments, the catheter comprises a connector so as to be suitable for connection to a flow control device to be provided. The connector may be a Luer connector or other suitable arrangement.

In some embodiments, the catheter comprises a return end or a pigtail configuration.

As the skilled person will be aware, a pigtail configuration refers to a catheter having a return end or coiled end. Various catheters comprising a pigtail configuration are available commercially and are known in the art. Pigtail configurations assist in holding the catheter in place, for instance once inserted into the patient. A pigtail configuration may also assist in allowing the flow of fluid injected through the catheter, for example fluid for imaging studies. Furthermore, a pigtail configuration is less susceptible to occlusion of the catheter tip with blood or blood clots.

In some embodiments, the assembly further comprises a removable guide wire of sufficient length to extend through the vascular access tube, for guiding the catheter.

It is understood that the expression "guide wire" is a known functional term and may refer to any structure that is sufficiently rigid to assist with the deployment of the catheter in situ. It is not necessary for the guide wire to be a metal wire. The guide wire may comprise metal.

In embodiments where the vascular access tube and/or access arm comprises a partition structure, the removable guide wire may extend through the first or the second passage as defined by the partition structure. This further assists in guiding the insertion of the catheter.

In embodiments wherein the cross-section of the first passage is greater than the cross-section of the second passage, the removable guide wire may extend through the second passage.

In some embodiments, the assembly further comprises a flow sensor configured to measure a flow value representative of a flow rate of fluid flowing through the catheter. In some embodiments, the flow sensor is integral with or attached to the catheter.

This allows the flow rate of the fluid to be measured as it is removed by the catheter. Suitable flow sensors will be known in the art. Example flow sensors include, but are not limited to, rotary potentiometer based sensors, heat sensors, velocimeters, Doppler-based or laser-based flow sensors.

In some embodiments, the catheter is connected to the flow control device. The flow sensor may be in communication with the flow control device. The flow sensor and the flow control device may be in communication such that the flow of the blood is controlled by a closed-loop feedback based mechanism.

For example, a controller may be set to a threshold flow value. In use, if the flow sensor detects a flow value below the threshold value, this can be communicated to the flow control device which can then increase flow. Alternatively, if the flow sensor detects a flow value above the threshold value, this can be communicated to the flow control device which can then decrease flow. This provides an accurate flow to ensure maximal removal of blood from the subject without damaging the subject.

The flow control device may be a suction-generating device such as a negative or low pressure source. In use, when connected to the catheter, the flow control device applies a negative or low pressure which creates a driving pressure to remove the blood from the left ventricular cavity using the catheter.

The flow control device may be an arterial pump, a roller pump, a vacuum source, a perfusion pump or a motive-venturi type apparatus.

The methods described in the following do not fall under the claimed invention. According to a second aspect not falling under the claimed invention, there is provided a method. The method is a method of accessing the luminal side of a blood vessel through the wall of a vascular access tube comprising an access port in accordance with any one of the preceding embodiments. The method comprises the steps of attaching the vascular access tube to the blood vessel, traversing the access port with an object such as a catheter, and inserting the object through the access port beyond a distal end of the vascular access tube.

By utilising an access port in accordance with any one of the preceding embodiments, the method allows maintaining fluid subject to a driving pressure of at least 50 mmHg (0.0658 atm) within the vascular access tube, whether or not an object to be provided (such as a catheter) is inserted through the access port.

In some embodiments, the method comprises the steps of using a guide wire to insert the object through the access port and beyond the distal end of the vascular access tube, and withdrawing the guide wire.

In some embodiments, the object is a catheter, and the method comprises using a flow control device to apply a driving pressure to the catheter to withdraw fluid via a distal end of the catheter. Herein, the catheter driving pressure (the suction applied to the catheter) will be understood to be controlled separately from the main lumen driving pressure (the driving pressure applied to fluid through the vascular access tube).

In some embodiments, the method comprises the steps of using a flow sensor to measure a flow value representative of the flow rate of fluid flowing through the catheter, and modulating flow control parameters of the flow control device in response to the flow value.

In some embodiments, the method comprises the step of removing the object from the vascular access tube.

In some embodiments, the method comprises the step of re-inserting the object through the access port beyond a distal end of the vascular access tube.

In some embodiments, the method comprises the step of manually shutting the access port.

Embodiments of the second aspect relate to using the embodiments of the first aspect. As such, any embodiments and features described in relation to the first aspect may be used in embodiments of the second aspect.

The method may be used for medical research not involving the human or animal body, for instance for flow studies on phantoms or test setups.

### Summary of the Figures

The present invention will now be described by way of example and with reference to the following figures:
Figure 1A shows a schematic side view of a vascular access tube in accordance with an embodiment;
Figure 1B shows the Figure 1A embodiment in an assembly with an additional component;
Figure 2 shows a cross-sectional view across the line X-X of the tube of Figure 1B;
Figure 3 shows a cross-sectional view of a vascular access tube in accordance with another embodiment;
Figure 4 shows a schematic illustration of the Figure 1B embodiment in a blood removal system; and
Figure 5 shows steps of a method of using a vascular access tube in accordance with an embodiment.

### Detailed Description

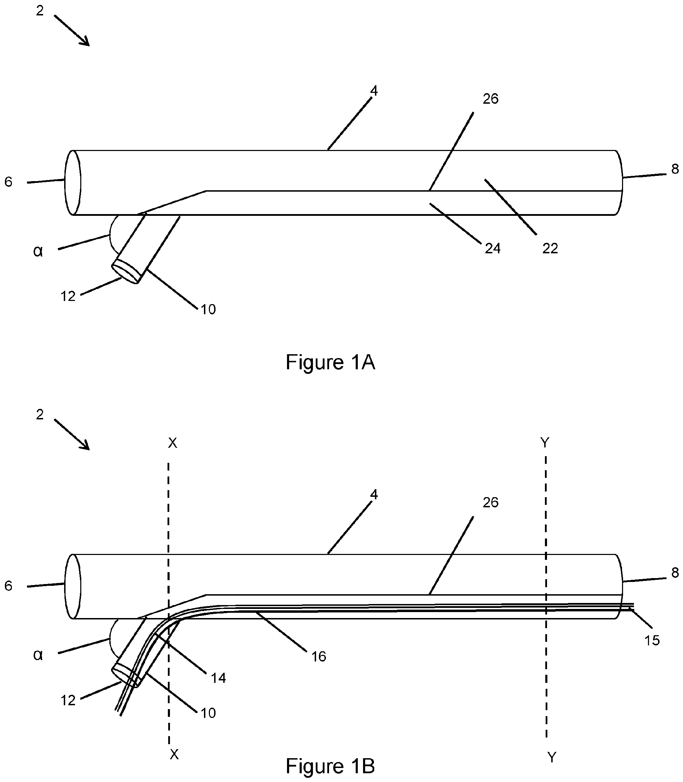

Figure 1A shows a side view of a vascular access tube 2. The vascular access tube comprises a wall 4 which has a generally tubular cross-section defining a main lumen. The tube 2 has a first end 6 and a second end 8. Extending from an outer side of the wall 4 of the tube 2 is an access arm 10 that is tubular and which provides the access port of the tube. The access arm 10 has a first end connected to the wall 4 of the vascular tube, such that the lumen of the access arm 10 is in fluid communication with the lumen of the vascular access tube 2. A second end of the access arm 10 is distal to the lumen of the vascular access tube and constitutes a free end. In the absence of any barriers, the free end of the access arm 10 provides a passage into the main lumen of the vascular access tube 2 through the wall 4, without requiring access from either the first end 6 or the second end 8. The fluid passage leads from the distal end through the access arm 10 and through the first end through an aperture in the wall 4 where the access arm joins the main lumen, and into the vascular access tube 2.

The access arm 10 extends at an angle alpha (α) from the wall 4. In the present embodiment angle α is approximately 45°, but other angles can be envisaged. The vascular access tube 2 and the access arm 10 may be flexible and so the angle α may vary in practice.

The access arm 10 extends from a portion of the wall 4 closer to the first end 6 than the second end 8, such that the access port, in this embodiment the access arm 10, is positioned off-centre.

Also shown in Figure 1A is a collapsible wall 26 extending part-way along the main lumen and separating the main lumen into a first passage 22 and a second passage 24. The collapsible wall 26 is optional.

A self-sealing membrane 12 providing a biasing structure with self-closing behaviour is located at the free end of the access arm 10. It will be appreciated that in other embodiments, the self-sealing membrane 12 can be positioned to reversibly close the end of the access arm 10 proximal to the vascular access tube 2, i.e. at or near to the junction between the access arm 10 and the vascular access tube 2. Alternatively, the membrane 12 can be positioned at any site along the length of the access arm10. In embodiments without access arm 10 the self-sealing membrane 12 may be directly on the wall 4.

Instead of a self-sealing membrane 12, other suitable structures may be provided, such as a valve, such as a haemostatic valve. The self-sealing property of the membrane 12 allows it to be punctured, for instance with a catheter to be provided, while remaining fluid-tight. This allows the main lumen to be accessed when fluid at driving pressures up to 50 mmHg or higher is flowing through the main lumen.

Figure 1B schematically shows the vascular access tube 2 of Figure 1A with a catheter 14 inserted through the membrane 12 with the help of a guide wire 16. It will be appreciated that an object other than the catheter 14 may be inserted into the lumen. With the catheter 14 (or other object) inserted, the self-sealing properties of the membrane 12 cause the membrane 12 to draw together around the catheter 14 and thereby provide a seal around the a catheter 14 that is sufficiently fluid-tight to reduce, and practically prevent, the risk of pressurised fluid spraying from the main lumen toward the outside. In practice, a small amount of leakage may be tolerable. The membrane 12 practically avoids any spraying of pressurised blood flowing through the tube 2 that would be observed when piercing the wall 4. Once the catheter 14 (or other object) is removed, the self-sealing membrane 12 closes to seal the aperture so that the membrane is sufficiently fluid-tight to maintain the sealed characteristic of the wall 4. Seals and valve structures (such as duck-bill valves) are known in the art that are sufficiently fluid-tight to prevent fluid loss particularly of blood flowing at pressures and at flow rates typically expected in a patient during surgery.

Figure 1B shows the catheter 14 inserted into the self-sealing membrane 12, through the access arm 10, through the second passage 24 of the main lumen, and through and beyond the second end 8. In Figure 1B, a distal end 15 of the catheter 14 protrudes outside the second end 8 to indicate that the distal end 15 protrudes beyond the end of the vascular access tube 2. It will be understood that a suitably long catheter may extend much further beyond the vascular access tube. As such, with a sufficiently long catheter 14, practically any point of the lumen of the vascular access tube 2, as well as a region beyond the vascular access tube 2 can be accessed by the catheter 14. This is achieved without requiring access through the first end 6 of the vascular access tube 2. It will be appreciated that the membrane 12 continues to maintain a fluid-tight seal as it gathers around the catheter 14, while a fluid passage into and out of the lumen from the access port is provided via the catheter 14.

In the present embodiment the access arm 10 is a separate component to the vascular access tube 2, the access arm 10 having being joined to the vascular access tube such that the lumen of the access arm 10 is in fluid communication with the lumen of the vascular access tube 2. However, it will be appreciated that the access arm 10 may be integral with the vascular access tube 2.

As shown in Figures 1A and 1B, the diameter of the access arm 10 is smaller than the diameter of the main passage of the vascular access tube. It will, however, be appreciated that other diameters and diameter differences can be envisaged. For instance, the access arm 10 may have the same diameter as the vascular access tube.

In the present embodiment, the access arm 10 and the vascular access tube are formed of the same flexible material. A suitable flexible material is dacron. In other embodiments, the access arm 10 and the vascular access tube may be formed of different flexible material, both being formed of the same material or different.

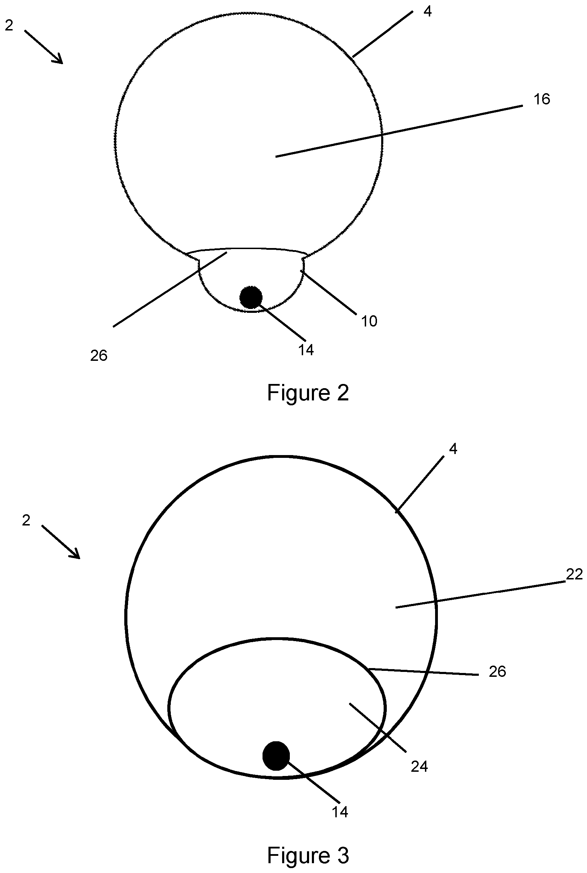

Figure 2 shows a cross-sectional view across the line X-X of the tube of Figure 1B, in a plane perpendicular to the axis of the main lumen, approximately at the joint of the access arm 10 with the vascular access tube 2. The same numerals are used in Figure 2 as for corresponding elements in Figure 1 and the description of corresponding parts is not necessarily repeated. Figure 2 also shows, in section, the catheter 14 which extends (through the plane of the section of Figure 2) along the access arm 10 and within the collapsible wall 26 of the vascular access tube 2.

Figure 3 shows a cross-section across the line Y-Y of the tube of Figure 1B. The same numerals are used in Figure 3 for corresponding structures in the other Figures. The Figure 3 cross section is taken further along of the main lumen of the vascular access tube 2 and does not show the access arm 10. Figure 3 shows more clearly that the lumen 16 of the vascular access tube 20 is separated into a first passage 22 and a second passage 24 by an internal partition structure, in this embodiment a collapsible wall 26. The first passage 22 has a greater cross-section than the second passage 24, e.g. at a ratio 1:2 or 1:3. Other suitable ratios between the cross-sections may be used.

Only the second passage 24 is in fluid communication with the access arm 10 (not shown in Figure 3). The first passage 22 is fluidly isolated from the access arm due to the internal partition structure. As such, the catheter 14 is not exposed to fluid flowing through the first passage 22. It will be understood that the first passage 22 may or may not be fluidly isolated depending on the wall structures defining the first and second passages 22, 24. In some embodiments, no internal partition structure is provided.

The smaller second passage 24 serves as a guide channel for the catheter 14. The second passage 24 may be sealed against the first end 6 such that no fluid from the first end 6 enters the second passage 24. Thereby, any object inserted into the second passage is not directly exposed to the fluid in the first passage 22.

If no object is inserted in to the second passage 24 then the collapsible wall 26 may collapse, due to the fluid pressure in the first passage 22, against the inner surface of the wall 4. In that case, the cross-section of the first passage 22 corresponds practically to the cross-section of the wall 4.

In use, the vascular access tube 2 is attached (anastomosed) with the second end 8 into or onto the vessel of a subject, such that the second end 8 is implanted into or onto the vessel of the subject. A portion of the access arm 10 may remain external to the subject.

In the absence of a catheter 14, the vascular access tube 2 provides a passage from the first end 6 to the second end 8 whose main lumen is practically fluid isolated from the outside. The access arm 10 is sealed by the seal 12 in a sufficiently fluid-tight manner to maintain the fluid isolation of the vascular access tube 2.

If, after implantation of the vascular access tube 2, it is necessary to have access to the patient's vessel, this can be achieved as follows. The first and second ends 6, 8 may no longer be accessible from the outside. Furthermore, it may not be practical to open the wall 4 of the vascular access tube 2, because it may be practically impossible to restore its fluid-tight property. This is the case particularly for arterial access grafts, as these have to withstand high driving pressure.

However, by way of the self-sealing membrane 12, a mechanism is provided to allow access into the main lumen of the vascular access tube and/or to organs beyond the second end 8. The self-sealing membrane 12 can be traversed by an object suitable for the procedure, for example, the catheter 14 as shown in Figure 1B. The catheter 14 is inserted through the self-sealing membrane 12, into the access arm 10 and further pushed into the vascular access tube such that the catheter 14 extends beyond the second end 8 of the vascular access tube and into the vessel of the patient.

The self-sealing nature of the membrane ensures that the membrane maintains a fluid-tight seal with the catheter 14 such that fluid access to the lumen of the tube is possible via the catheter 14.

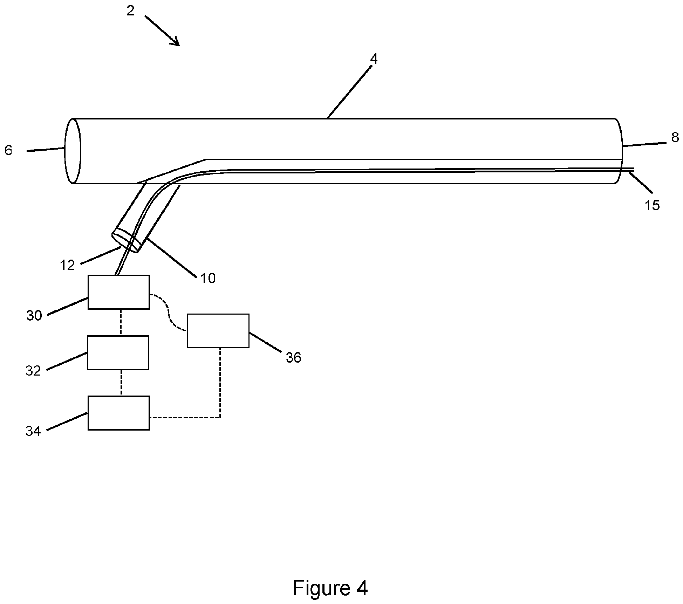

Figure 4 shows the device of Figure 1B as part of a blood removal system, with the catheter 14 introduced into the vascular access tube 2. The same numerals are used in Figure 4 for corresponding elements in the preceding Figures. In Figure 4, the guide wire (cf. Figure 1B) has been removed.

At the outer (distal) end of the catheter 14, there is a connector 30 (illustrated schematically) for connection to a flow control device 32 (illustrated schematically). The catheter 14 may also comprise an integral flow sensor 36 (illustrated schematically) or a flow sensor that is operationally linked with the flow path through the catheter 14. Prior to or after insertion of the catheter 14, the connector 30 is inserted to or operationally linked to the flow control device 32, for example an arterial pump. The pump may be a roller pump or a centrifugal pump, to name two examples. The flow control device 32 may be a controller of a low-pressure or "vacuum"-induced flow mechanism.

Once the catheter is introduced into the vessel of the patient and the flow control device 32 is connected, the flow control device 32 applies a negative pressure to the catheter 14. This generates suction which serves to pull blood out of the vessel, or an area such as the left ventricle, of the patient into and through the catheter 14 so that the blood can be removed. This is especially beneficial in left ventricular assist procedures, where a build-up of stagnant or clotted blood in the left ventricle of the subject can, otherwise, risk complications, and which needs to be carried out while the heart continuously supplied by oxygenated blood at the necessary driving pressures.

As the blood flows through the catheter, the flow rate may be measured by the integral flow sensor 36. The flow rate may be provided to a controller 34 (illustrated schematically) as an input. This may be provided as part of a closed loop control. For instance, to maintain the blood flow at a pre-determined threshold, if the flow rate is below the pre-determined threshold the flow control device 32 may be controlled by the controller 34 to increase the negative pressure (such that a stronger suction is applied). If the flow rate is above the pre-determined threshold the flow control device 32 may be controlled by the controller 34 to decrease the negative pressure (such that a weaker suction is applied).

As such, the controller 34 may include a control loop allowing the flow rate through the access arm 10 to be modulated in response to temporary fluctuations. The control loop allows the flow through the catheter to be maintained at a pre-determined level.

This allows a closed-loop control to be provided of the amount of blood removed from the left ventricular cavity. For instance, in practice, a clinician may determine a suitable flow rate target level at which blood should be continuously removed from the left ventricular cavity. The flow rate target level may be low to reduce any stress on the patient by maintaining a steady removal of fluid.

A processor of the controller 34 may determine the volume of blood removed from the left ventricular cavity, either from deriving this from a flow sensor 36 or from a reservoir into which the blood is collected. The amount of blood in a reservoir may be determined by a level sensor. The volume of blood may be indicative of aortic valve condition. The processor may determine the total volume of blood removed during a procedure or the volume of blood removed per unit time.

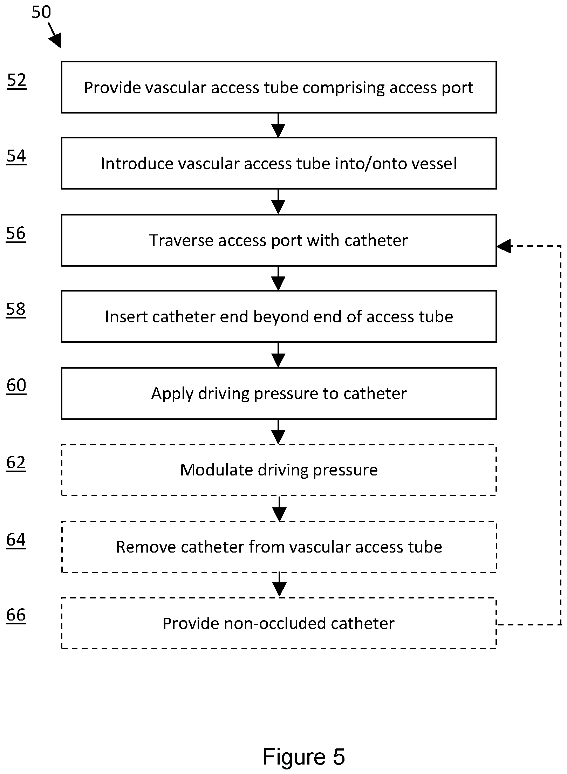

Figure 5 shows steps of a method 50 (not falling under the claimed invention) of using a vascular access tube. In step 52, an arterial access tube, such as vascular access tube 2, is provided. The vascular access tube comprises a self-sealing membrane 12 or a valve to provide an access port. In step 54, the arterial access tube is inserted into a blood vessel. This step may involve implanting on anastomosing the arterial access tube onto a blood vessel. In step 56, a catheter 14 is introduced via the access port into the arterial access tube. In step 58, an end of the catheter is introduced beyond an end of the arterial access tube. In step 60, a driving pressure is applied to the catheter. The driving pressure is sufficiently strong to suck fluid from the distal catheter tip to remove fluid. This procedure may be used to remove stagnant blood from the left ventricular cavity. In an optional step 62, the driving pressure is modulated, for instance in response to a flow sensor measurement, to ensure the flow rate through the catheter is close to or at a target flow value. In an optional step 64, the catheter is removed from the arterial access tube. This may be necessary due to an occlusion of the catheter. In optional step 66, a non-occluded catheter is provided. This may involve removing an occlusion from the catheter. This may involve providing a new catheter. The method may be continued at step 56 by inserting the non-occluded catheter via the access port into the arterial access tube.

The vascular access tube of the invention, once implanted or attached, provides repeated access via the walls of the vascular access tube without having to open up other portions of the body. For instance, even during routine blood-removal operation with a pigtail catheter, it is possible that the catheter tip is occluded by blood or blood clots. In that event, the present vascular access tube allows removal of the catheter, cleaning or replacing the catheter, and inserting a non-occluded catheter to continue with the blood removal procedure. This catheter replacement may be carried out while blood is flowing through the arterial access graft at the required driving pressure. As such, the access port of the invention may be used for other procedures.

## Claims

1. An arterial access graft (2) for blood subject to a driving pressure, the arterial access graft being configured for surgical attachment to an axillary artery and for providing access to the axillary artery via an access port, the arterial access graft comprising a wall (4) defining a main lumen, wherein a portion of the wall comprises the access port for an object to be introduced into the graft through the wall, wherein the access port comprises a biasing structure providing a self-closing behaviour sufficiently strong to remain fluid-tight when exposed to a driving pressure of up to 200 mmHg (0.263 atm) or higher, whereby the access port is sufficiently fluid-tight to contain pressurised blood flowing through the arterial access graft.

2. The arterial access graft (2) according to claim 1, wherein the biasing structure comprises a self-sealing membrane (12) and/or a valve.

3. The arterial access graft (2) according to claim 1 or 2, wherein the self-closing behaviour of the biasing structure is stronger than that of the wall (4).

4. The arterial access graft (2) according to any preceding claim, wherein the access port comprises an access arm (10) comprising an access lumen, the access arm extending from the wall (4), and optionally, wherein the access lumen has a smaller diameter than the main lumen.

5. The arterial access graft (2) according to claim 4, wherein the biasing structure is positioned to seal an end of the access arm (10) distal to the main lumen of the arterial access graft.

6. The arterial access graft (2) according to claim 4 or claim 5, wherein the access arm (10) extends at an angle of at least 10°, 20°, 30°, or at least 45°from the wall (4) of the arterial access graft (2).

7. The arterial access graft (2) according to any preceding claim, wherein the main lumen comprises a partition structure (26) extending along at least a portion of the length of the graft to separate the main lumen into at least two passages (22, 24), wherein, optionally, the cross-section of one of the passages (22) is greater than another passage (24).

8. The arterial access graft (2) according to claim 7, wherein the access port joins into one passage (24).

9. The arterial access graft (2) according to any preceding claim, wherein the arterial access graft is for facilitating removal of blood from a left ventricular cavity, and the access port is configured to receive a catheter (14) for removing blood from the left ventricular cavity.

10. The arterial access graft (2) according to any preceding claim, wherein the biasing structure provides a self-closing behaviour sufficiently strong to remain fluid-tight when exposed to a driving pressure of up to 400 mmHg (0.526 atm).

11. A blood removal assembly suitable for removing blood from a left ventricular cavity, the assembly comprising
an arterial access graft (2) as defined in any preceding claim, and
a catheter (14) dimensioned to fit through the access port of the arterial access graft (2), the catheter (14) being of sufficient length to extend from outside the access port beyond an end of the arterial access graft.

12. The blood removal assembly according to claim 11, wherein the catheter (14) comprises a connector (30) so as to be suitable for connection to a flow control device (32) to be provided, wherein, optionally, the connector is a Luer connector.

13. The blood removal assembly according to claim 11 or claim 12, wherein the assembly further comprises a removable guide wire (16) of sufficient length to extend through the arterial access graft (2), for guiding the catheter (14).

14. The blood removal assembly according to any one of claims 11 to 13, wherein the assembly further comprises a flow sensor (36) configured to measure a flow value representative of a flow rate of fluid flowing through the catheter (14).

15. The blood removal assembly according to claim 14, further comprising a controller (34) configured to obtain the flow value and to modulate flow control parameters of a flow control device (32) in response to the flow value.

## Patentansprüche

1. Arterielle Zugangsprothese (2) für Blut, das einem treibenden Druck ausgesetzt ist, wobei die arterielle Zugangsprothese für eine chirurgische Befestigung an einer Achselarterie und zum Bereitstellen eines Zugangs zu der Achselarterie über einen Zugangsanschluss konfiguriert ist, wobei die arterielle Zugangsprothese eine Wand (4) umfasst, die ein Hauptlumen definiert, wobei ein Abschnitt der Wand den Zugangsanschluss umfasst, damit ein Objekt durch die Wand in die Prothese eingeführt wird, wobei der Zugangsanschluss eine Vorspannungsstruktur umfasst, die ein selbstschließendes Verhalten gewährleistet, das ausreichend stark ist, um fluiddicht zu bleiben, wenn sie einem treibenden Druck von bis zu 200 mmHg (0,263 atm) oder höher ausgesetzt ist, wodurch der Zugangsanschluss ausreichend fluiddicht ist, um unter Druck stehendes Blut zu enthalten, das durch die arterielle Zugangsprothese strömt.

2. Arterielle Zugangsprothese (2) nach Anspruch 1, wobei die Vorspannungsstruktur eine selbstabdichtende Membran (12) und/oder ein Ventil umfasst.

3. Arterielle Zugangsprothese (2) nach Anspruch 1 oder 2, wobei das selbstschließende Verhalten der Vorspannungsstruktur stärker ist als dasjenige der Wand (4).

4. Arterielle Zugangsprothese (2) nach einem der vorhergehenden Ansprüche, wobei der Zugangsanschluss einen Zugangsarm (10) umfasst, der ein Zugangslumen umfasst, wobei sich der Zugangsarm von der Wand (4) aus erstreckt und wahlweise, wobei das Zugangslumen einen kleineren Durchmesser aufweist als das Hauptlumen.

5. Arterielle Zugangsprothese (2) nach Anspruch 4, wobei die Vorspannungsstruktur dafür positioniert ist, ein Ende des Zugangsarms (10), distal zu dem Hauptlumen der arteriellen Zugangsprothese, abzudichten.

6. Arterielle Zugangsprothese (2) nach Anspruch 4 oder Anspruch 5, wobei sich der Zugangsarm (10) in einem Winkel von mindestens 10°, 20°, 30° oder mindestens 45° von der Wand (4) der arteriellen Zugangsprothese (2) aus erstreckt.

7. Arterielle Zugangsprothese (2) nach einem der vorhergehenden Ansprüche, wobei das Hauptlumen eine Teilungsstruktur (26) umfasst, die sich entlang mindestens eines Abschnitts der Länge der Prothese erstreckt, um das Hauptlumen in mindestens zwei Durchgänge (22, 24) zu trennen, wobei, wahlweise, der Querschnitt eines der Durchgänge (22) größer ist als der eines anderen Durchgangs (24).

8. Arterielle Zugangsprothese (2) nach Anspruch 7, wobei der Zugangsanschluss in einen Durchgang (24) mündet.

9. Arterielle Zugangsprothese (2) nach einem der vorhergehenden Ansprüche, wobei die arterielle Zugangsprothese zum Erleichtern des Entnehmens von Blut aus einer linken Herzkammer dient und der Zugangsanschluss dafür konfiguriert ist, einen Katheter (14) zum Entnehmen von Blut aus der linken Herzkammer aufzunehmen.

10. Arterielle Zugangsprothese (2) nach einem der vorhergehenden Ansprüche, wobei die Vorspannungsstruktur ein selbstschließendes Verhalten gewährleistet, das ausreichend stark ist, um fluiddicht zu bleiben, wenn sie einem treibenden Druck von bis zu 400 mmHg (0,526 atm) ausgesetzt ist.

11. Blutentnahmebaugruppe, geeignet zum Entnehmen von Blut aus einer linken Herzkammer, wobei die Baugruppe Folgendes umfasst
eine arterielle Zugangsprothese (2) nach einem der vorhergehenden Ansprüche, und
einen Katheter (14), der bemessen ist, um durch den Zugangsanschluss der arteriellen Zugangsprothese (2) zu passen, wobei der Katheter (14) eine ausreichende Länge hat, um sich von außerhalb des Zugangsanschlusses über ein Ende der arteriellen Zugangsprothese hinaus zu erstrecken.

12. Blutentnahmebaugruppe nach Anspruch 11, wobei der Katheter (14) einen Verbinder (30) umfasst, um so für eine Verbindung mit einer bereitzustellenden Durchflussregelungseinrichtung (32) geeignet zu sein, wobei, wahlweise, der Verbinder ein Luer-Verbinder ist.

13. Blutentnahmebaugruppe nach Anspruch 11 oder Anspruch 12, wobei die Baugruppe ferner einen entfernbaren Führungsdraht (16) mit einer ausreichenden Länge, damit er sich durch die arterielle Zugangsprothese (2) erstreckt, zum Führen des Katheters (14), umfasst.

14. Blutentnahmebaugruppe nach einem der Ansprüche 11 bis 13, wobei die Baugruppe ferner einen Durchflusssensor (36) umfasst, der dafür konfiguriert ist, einen Durchflusswert zu messen, der eine Durchflussmenge eines Fluids, das durch den Katheter (14) strömt, darstellt.

15. Blutentnahmebaugruppe nach Anspruch 14, die ferner einen Regler (34) umfasst, der dafür konfiguriert ist, den Durchflusswert zu erhalten und als Reaktion auf den Durchflusswert Durchflussregelungsparameter einer Durchflussregelungseinrichtung (32) zu modulieren.

## Revendications

1. Greffon d'accès artériel (2) pour du sang soumis à une pression d'entraînement, le greffon d'accès artériel étant configuré pour une liaison chirurgicale sur une artère axillaire et pour assurer un accès à l'artère axillaire via un orifice d'accès, le greffon d'accès artériel comprenant une paroi (4) définissant une lumière principale, dans lequel une partie de la paroi comprend l'orifice d'accès pour qu'un objet soit introduit à l'intérieur du greffon au travers de la paroi, dans lequel l'orifice d'accès comprend une structure de sollicitation assurant un comportement d'auto-fermeture suffisamment fort pour lui permettre de rester étanche aux fluides lorsqu'elle est exposée à une pression d'entraînement allant jusqu'à 200 mmHg (0,263 atm) ou plus, moyennant quoi l'orifice d'accès est suffisamment étanche aux fluides pour contenir du sang sous pression qui s'écoule au travers du greffon d'accès artériel.

2. Greffon d'accès artériel (2) selon la revendication 1, dans lequel la structure de sollicitation comprend une membrane auto-étanchéifiante (12) et/ou une soupape.

3. Greffon d'accès artériel (2) selon la revendication 1 ou la revendication 2, dans lequel le comportement d'auto-fermeture de la structure de sollicitation est plus fort que celui de la paroi (4).

4. Greffon d'accès artériel (2) selon l'une quelconque des revendications précédentes, dans lequel l'orifice d'accès comprend un bras d'accès (10) comprenant une lumière d'accès, le bras d'accès s'étendant depuis la paroi (4), et optionnellement, dans lequel la lumière d'accès présente un diamètre plus petit que la lumière principale.

5. Greffon d'accès artériel (2) selon la revendication 4, dans lequel la structure de sollicitation est positionnée pour assurer l'étanchéité d'une extrémité du bras d'accès (10) qui est distale par rapport à la lumière principale du greffon d'accès artériel.

6. Greffon d'accès artériel (2) selon la revendication 4 ou la revendication 5, dans lequel le bras d'accès (10) s'étend selon un angle d'au moins 10°, 20°, 30°, ou d'au moins 45° par rapport à la paroi (4) du greffon d'accès artériel (2).

7. Greffon d'accès artériel (2) selon l'une quelconque des revendications précédentes, dans lequel la lumière principale comprend une structure de partition (26) s'étendant le long d'au moins une partie de la longueur du greffon pour séparer la lumière principale en au moins deux passages (22, 24), dans lequel, optionnellement, la section en coupe transversale de l'un des passages (22) est plus grande que celle d'un autre passage (24).

8. Greffon d'accès artériel (2) selon la revendication 7, dans lequel l'orifice d'accès se joint à un passage (24).

9. Greffon d'accès artériel (2) selon l'une quelconque des revendications précédentes, dans lequel le greffon d'accès artériel a pour objet de faciliter le retrait de sang depuis une cavité ventriculaire gauche, et l'orifice d'accès est configuré pour recevoir un cathéter (14) pour retirer du sang depuis la cavité ventriculaire gauche.

10. Greffon d'accès artériel (2) selon l'une quelconque des revendications précédentes, dans lequel la structure de sollicitation assure un comportement d'auto-fermeture suffisamment fort pour lui permettre de rester étanche aux fluides lorsqu'elle est exposée à une pression d'entraînement allant jusqu'à 400 mmHg (0,526 atm).

11. Ensemble de retrait de sang approprié pour retirer du sang depuis une cavité ventriculaire gauche, l'ensemble comprenant
un greffon d'accès artériel (2) selon l'une quelconque des revendications précédentes, et
un cathéter (14) dimensionné pour être ajusté au travers de l'orifice d'accès du greffon d'accès artériel (2), le cathéter (14) présentant une longueur suffisante pour s'étendre depuis l'extérieur de l'orifice d'accès au-delà d'une extrémité du greffon d'accès artériel.

12. Ensemble de retrait de sang selon la revendication 11, dans lequel le cathéter (14) comprend un connecteur (30) afin d'être approprié pour une connexion sur un dispositif de commande d'écoulement (32) à prévoir, dans lequel, optionnellement, le connecteur est un connecteur Luer.

13. Ensemble de retrait de sang selon la revendication 11 ou la revendication 12, dans lequel l'ensemble comprend en outre un fil de guidage amovible (16) d'une longueur suffisante pour s'étendre au travers du greffon d'accès artériel (2), pour guider le cathéter (14).

14. Ensemble de retrait de sang selon l'une quelconque des revendications 11 à 13, dans lequel l'ensemble comprend en outre un capteur d'écoulement (36) configuré pour mesurer une valeur d'écoulement représentative d'un débit d'écoulement d'un fluide s'écoulant à travers le cathéter (14).

15. Ensemble de retrait de sang selon la revendication 14, comprenant en outre un dispositif de commande (34) configuré pour obtenir la valeur d' écoulement et pour moduler des paramètres de commande d'écoulement d'un dispositif de commande d'écoulement (32) en réponse à la valeur d'écoulement.
